(19) Europäisches Patentamt
European Patent Office
Office européen des brevets

(11) Veröffentlichungsnummer: **0 594 963 A1**

(12) # EUROPÄISCHE PATENTANMELDUNG

(21) Anmeldenummer: **93113475.3**

(22) Anmeldetag: **24.08.93**

(51) Int. Cl.[5]: **C07D 213/30**, C07D 213/32, C07D 213/34, C07D 213/26, A01N 43/40

(30) Priorität: **04.09.92 DE 4229643**

(43) Veröffentlichungstag der Anmeldung: **04.05.94 Patentblatt 94/18**

(84) Benannte Vertragsstaaten: **BE CH DE ES FR GB IT LI NL**

(71) Anmelder: **BAYER AG**
**D-51368 Leverkusen(DE)**

(72) Erfinder: **Elbe, Hans-Ludwig, Dr.**
**Dasnöckel 59**
**D-42329 Wuppertal(DE)**
Erfinder: **Böhm, Stefan, Dr.**
**Andreas-Gryphius-Strasse 9**
**D-51065 Köln(DE)**
Erfinder: **Tiemann, Ralf, Dr.**
**Ernst-Ludwig-Kirchner-Strasse 5**
**D-51375 Leverkusen(DE)**
Erfinder: **Dutzmann, Stefan, Dr.**
**Kosenberg 10**
**D-40721 Hilden(DE)**
Erfinder: **Dehne, Heinz-Wilhelm, Dr.**
**Krischer Strasse 81**
**D-40789 Monheim(DE)**

(54) **Substituierte Hydroxyalkylpyridine.**

(57) Neue substituierte Hydroxyalkylpyridine der Formel (I),

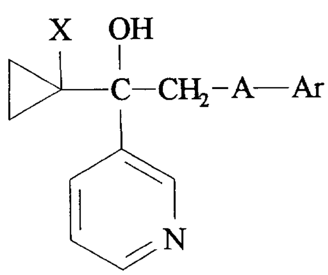

(I)

in welcher
X, A und Ar die in der Beschreibung gegebenen Bedeutungen haben sowie deren Säureadditions-Salze und Metallsalz-Komplexe und ihre Verwendung zur Bekämpfung von Schädlingen, vor allem auf dem Pflanzenschutzgebiet und im Materialschutz.

Die Verbindungen der Formel (I) können nach bekannten Verfahren hergestellt werden, so z.B. aus geeigneten Cyclopropylketonen und metallorganischen Pyridin-Verbindungen.

EP 0 594 963 A1

Die Erfindung betrifft neue substituierte Hydroxyalkylpyridine, ein Verfahren zu ihrer Herstellung und ihre Verwendung in Schädlingsbekämpfungsmitteln.

Es ist bekannt, daß bestimmte substituierte Hydroxyalkylpyridine wie beispielsweise die Verbindung 4-(4-Chlorphenoxy)-2,2-dimethyl-3-hydroxy-1-phenyl-3-(3-pyridyl)-butan fungizide Eigenschaften besitzen (vergl. z.B. EP 302 366).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue substituierte Hydroxyalkylpyridine der Formel (I),

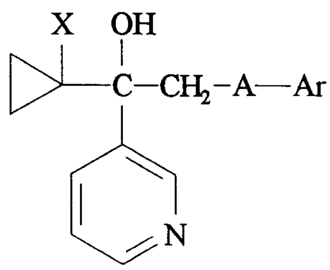

(I)

in welcher

X für Halogen steht,
A für Sauerstoff oder Schwefel steht und
Ar für gegebenenfalls substituiertes Aryl steht,

sowie deren Säureadditions-Salze oder Metallsalz-Komplexe gefunden.

Die Verbindungen der Formel (I) enthalten mindestens ein asymmetrisch substituiertes Kohlenstoffatom und können deshalb als optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden daß man die neuen substituierten Hydroxyalkylpyridine der Formel (I)

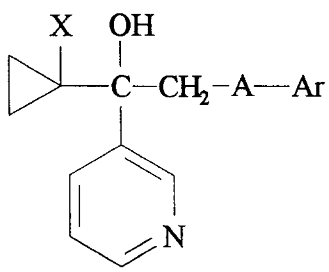

(I)

in welcher

X für Halogen steht,
A für Sauerstoff oder Schwefel steht und
Ar für gegebenenfalls substituiertes Aryl steht,

sowie deren Säureadditions-Salze oder Metallsalz-Komplexe erhält, wenn man Cyclopropylketone der Formel (II),

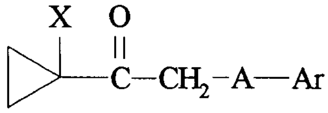

(II)

in welcher

X, A und Ar die oben angegebenen Bedeutungen haben,

mit metallorganischen Pyridin-Verbindungen der Formel (III),

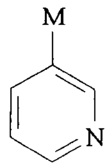

(III)

in welcher

M für ein Lithiumatom oder für eine Magnesium-Halogen-Gruppierung steht,

gegebenenfalls in Gegenwart eines Verdünnungsmittels umsetzt und gegebenenfalls an die so erhaltenen Verbindungen der Formel (I) eine Säure oder ein Metallsalz addiert.

Schließlich wurde gefunden, daß die neuen substituierten Hydroxyalkylpyridine der Formel (I) gute Wirksamkeit gegenüber Schädlingen besitzen.

Überrraschenderweise zeigen die erfindungsgemäßen substituierten Hydroxyalkylpyridine der allgemeinen Formel (I) eine erheblich bessere Wirksamkeit gegenüber pflanzenschädigenden Mikroorganismen im Vergleich zu den aus dem Stand der Technik bekannten substituierten Hydroxyalkylpyridine, wie beispielsweise die Verbindung 4-(4-Chlorphenoxy)-2,2-dimethyl-3-hydroxy-1-phenyl-3-(3-pyridyl)-butan, welches chemisch und/oder wirkungsmäßig naheliegende Verbindungen sind.

Die erfindungsgemäßen substituierten Hydroxyalkylpyridine sind durch die Formel (I) allgemein definiert. Bevorzugt sind Verbindungen der Formel (I), bei welchen

X für Fluor, Chlor, Brom oder Iod steht,

A für Sauerstoff oder Schwefel steht und

Ar für gegebenenfalls einfach oder mehrfach, gleich oder verschieden substituiertes Aryl mit 6 bis 10 Kohlenstoffatomen steht, wobei als Substituenten jeweils infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, Cycloalkyl mit 3 bis 8 Kohlenstoffatomen sowie gegebenenfalls einfach bis mehrfach, gleich oder verschieden durch Halogen, geradkettiges oder verzweigtes Alkyl, geradkettiges oder verzweigtes Alkoxy, geradkettiges oder verzweigtes Halogenalkyl und/oder geradkettiges oder verzweigtes Halogenalkoxy mit jeweils 1 bis 4 Kohlenstoffatomen und gegebenenfalls 1 bis 9 gleichen oder verschiedenen Halogenatomen substituiertes Phenyl.

Besonders bevorzugt sind Verbindungen der Formel (I), bei welchen

X für Fluor, Chlor oder Brom steht,

A für Sauerstoff oder Schwefel steht und

Ar für gegebenenfalls einfach bis fünffach, gleich oder verschieden substituiertes Aryl mit 6 oder 10 Kohlenstoffatomen z.B. Phenyl und Naphthyl, steht, wobei als Substituenten jeweils infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen, jeweils geradkettiges oder verzweigtes Alkoxycarbonyl oder Alkoximinoalkyl mit jeweils 1 bis 3 Kohlenstoffatomen in den einzelnen Alkylteilen, Cycloalkyl mit 3 bis 7 Kohlenstoffatomen sowie gegebenenfalls einfach bis fünffach, gleich oder verschieden durch Halogen, geradkettiges oder verzweigtes Alkyl, geradkettiges oder verzweigtes Alkoxy, geradkettiges oder verzweigtes Halogenalkyl und/oder geradkettiges oder verzweigtes Halogenalkoxy mit jeweils 1 bis 3 Kohlenstoffatomen und gegebenenfalls 1 bis 7 gleichen oder verschiedenen Halogenatomen substituiertes Phenyl.

Ganz besonders bevorzugt sind Verbindungen der Formel (I), bei welchen

X für Fluor oder Chlor steht,

A für Sauerstoff oder Schwefel steht und

Ar für gegebenenfalls einfach bis fünffach, gleich oder verschieden substituiertes Phenyl steht, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy,

Ethoxy, n- oder i-Propoxy, n-, i-, s- oder t-Butoxy, Methylthio, Ethylthio, Methylsulfinyl, Ethylsulfinyl, Methylsulfonyl, Ethylsulfonyl, Trifluormethyl, Difluormethyl, Trifluormethoxy, Difluormethoxy, Trifluormethylthio, Trifluormethylsulfinyl, Trifluormethylsulfonyl, Methoxycarbonyl, Ethoxycarbonyl, Methoximinomethyl, Methoximinoethyl, Ethoximinomethyl, Ethoximinoethyl, Cyclopropyl, Cyclopentyl, Cyclohexyl oder gegebenenfalls einbis dreifach, gleich oder verschieden durch Fluor, Chlor, Brom, Methyl, Ethyl, n- oder i-Propyl, Methoxy, Ethoxy, Trifluormethyl und/oder Trifluormethoxy substituiertes Phenyl.

Bevorzugte erfindungsgemäße Stoffe sind auch Additionsprodukte aus Säuren und denjenigen substituierten Hydroxyalkylpyridinen der Formel (I), in denen X, A und Ar die oben als bevorzugt genannten Bedeutungen haben.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure, und außerdem auch Saccharin und Thiosaccharin.

Außerdem bevorzugte erfindungsgemäße Stoffe sind Additionsprodukte aus Salzen von Metallen der II. bis IV. Haupt- und der I. und II. sowie IV. bis VIII. Nebengruppe des Periodensystems der Elemente und substituierten Hydroxyalkylpyridinen der Formel (I), in denen X, A und Ar die oben als bevorzugt genannten Bedeutungen haben.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. Chlorwasserstoffsäure und die Bromwasserstoffsäure, ferner Phosphorsäure, Salpetersäure und Schwefelsäure.

Verwendet man beispielsweise 1-Chlorcyclopropyl-2-(2,4-dichlorphenoxy)-ethan-1-on und 3-Pyridyl-lithium als Ausgangsverbindungen, so läßt sich der Reaktionsablauf des erfindungsgemäßen Verfahrens durch das folgende Formelschema darstellen:

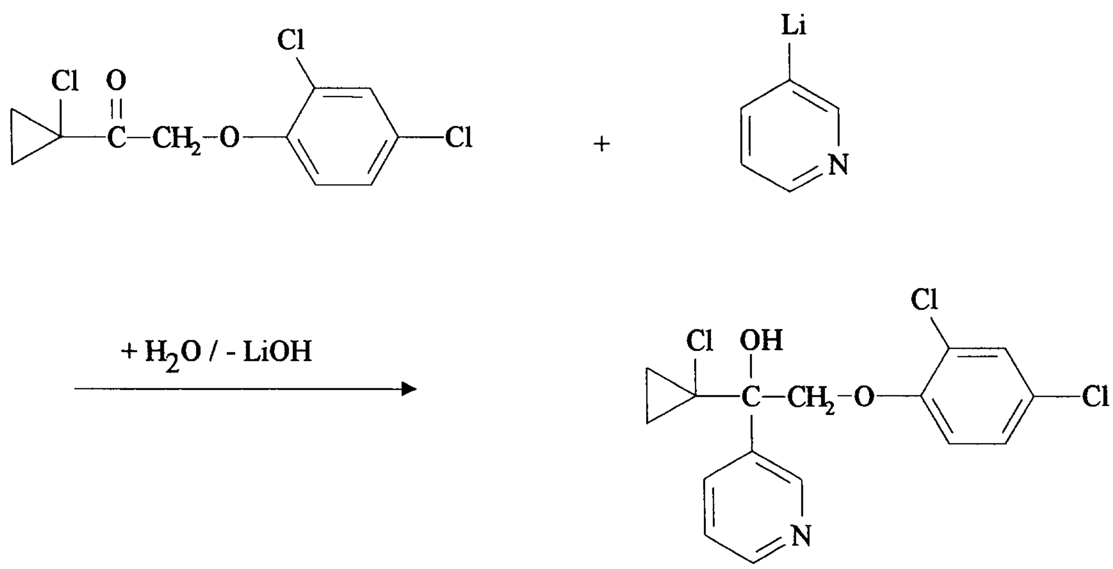


Die zur Durchführung des erfindungsgemäßen Verfahrens als Ausgangsstoffe benötigten Cyclopropylketone sind durch die Formel (II) allgemein definiert. In dieser Formel (II) stehen X, A und Ar vorzugsweise für diejenigen Reste, die bereits im Zusammenhang mit der Beschreibung der erfindungsgemäßen Verbindungen der Formel (I) als bevorzugt für diese Substituenten genannt wurden.

Die Cyclopropylketone der Formel (II) sind bekannt oder erhältlich in Analogie zu bekannten Verfahren (vergl. z.B. EP 297 383).

Die zur Durchführung des erfindungsgemäßen Verfahrens weiterhin als Ausgangsstoffe benötigten metallorganischen Pyridin-Verbindungen sind durch die Formel (III) allgemein definiert. In dieser Formel (III) steht M vorzugsweise für ein Lithiumatom oder für einen Mg-Cl- oder einen Mg-Br-Rest.

Die metallorganischen Pyridin-Verbindungen der Formel (III) sind allgemein bekannte Verbindungen der organischen Chemie.

Als Verdünnungsmittel zur Durchführung des erfindungsgemäßen Verfahrens kommen inerte organische Lösungsmittel infrage. Hierzu gehören insbesondere aliphatische, alicyclische oder aromatische Kohlenwasserstoffe, wie beispielsweise Benzin, Benzol, Toluol, Xylol, Petrolether, Hexan, Cyclohexan oder Ether, wie Diethylether, Diisopropylether, Dioxan, Tetrahydrofuran oder Ethylenglykoldimethyl- oder -diethylether oder Amide, wie Hexamethylphosphorsäuretriamid.

Die Reaktionstemperaturen können bei der Durchführung des erfindungsgemäßen Verfahrens in einem größeren Bereich variiert werden. Im allgemeinen arbeitet man bei Temperaturen zwischen -100 °C und +150 °C, vorzugsweise bei Temperaturen zwischen -80 °C und +120 °C.

Das erfindungsgemäße Verfahren wird üblicherweise unter Normaldruck durchgeführt. Es ist jedoch auch möglich unter erhöhtem oder vermindertem Druck zu arbeiten.

Zur Durchführung des erfindungsgemäßen Verfahrens setzt man pro Mol an Cyclopropylketon der Formel (II) im allgemeinen 1,0 bis 3,0 Mol, vorzugsweise 1,0 bis 1,5 Mol an metallorganischer Pyridin-Verbindung der Formel (III) ein. Die Reaktionsdurchführung, Aufarbeitung und Isolierung der Reaktionsprodukte erfolgt nach allgemein üblichen Methoden. Dabei kann die Umsetzung erforderlichenfalls in Gegenwart eines geeigneten Inertgases, wie beispielsweise Stickstoff oder Helium durchgeführt werden. Es ist auch möglich, die als Reaktionspartner einzusetzende metallorganische Pyridin-Verbindung der Formel (III) aus geeigneten Ausgangsverbindungen wie beispielsweise 3-Brompyridin und Isopropylmagnesiumbromid oder Butyllithium in einer vorgelagerten Reaktion direkt im Reaktionsgefäß herzustellen und ohne Isolierung anschließend im Eintopfverfahren mit den Cyclopropylketonen der Formel (II) gemäß dem erfindungsgemäßen Verfahren weiter umzusetzen (vergl. hierzu auch die Herstellungsbeispiele).

Die Reinigung der Endprodukte der Formel (I) erfolgt mit Hilfe üblicher Verfahren, beispielsweise durch Säulenchromatographie oder durch Umkristallisieren.

Die erfindungsgemäßen substituierten Hydroxyalkylpyridine der Formel (I) können in Säureadditions-Salze oder Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die Charakterisierung erfolgt mit Hilfe des Schmelzpunktes oder bei nicht kristallisierenden Verbindungen mit Hilfe des Brechungsindex oder der Protonen-Kernresonanzspektroskopie ($^1$H-NMR).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder Peronospora brassicae; Erysiphe-Arten, wie beispielsweise Erysiphe gramminis;
Sphaerotheca-Arten, wie beispielsweise Sphaerotheca fuliginea;

Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei können die erfindungsgemäßen Wirkstoffe mit besonders gutem Erfolg zur Bekämpfung von Getreidekrankheiten, wie beispielsweise gegen den Erreger der Netzfleckerkrankheit der Gerste (Pyrenophora teres) oder gegen den Erreger der Braunfleckenkrankheit an Gerste oder Weizen (Cochliobolus sativus) oder gegen den Erreger der Braunspelzigkeit des Weizens (Leptosphaeria nodorum) oder gegen den Erreger des echten Getreidemehltaues an Weizen oder Gerste (Erysiphe graminis), oder gegen den Erreger der Halmbruchkrankheit an Getreide (Pseudocercosporella herpotrichoides), gegen Fusariumarten oder zur Bekämpfung von Krankheiten im Obst- und Gemüseanbau, wie beispielsweise gegen den Erreger des Rebenmehltaues (Uncinula necator) oder gegen den Erreger des Apfelschorfes (Venturia inaequalis) oder gegen den Erreger des echten Gurkenmehltaues (Sphaerotheca fuliginea) oder gegen den Erreger des Apfelmehltaues (Podosphaera leucotricha) oder zur Bekämpfung von Reiskrankheiten wie beispielsweise gegen den Erreger der Reisfleckenkrankheit (Pyricularia oryzae) oder gegen den Erreger der Reisstengelkrankheit (Pellicularia sasakii) eingesetzt werden. Daneben besitzen die erfindungsgemäßen Wirkstoffe auch eine gute und breite in vitro-Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsuffoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende

Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akartzide und Herbizide sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die erfindungsgemäßen Wirkstoffe zeichnen sich neben der oben aufgeführten Wirksamkeit gegen pflanzenpathogene Mikroorganismen durch eine breite und starke mikrobizide Wirkung gegen ein breites Spektrum von für den Materialschutz relevanten Mikroorganismen aus sowie durch auffallend gute Wirksamkeit gegen Algen und Schleimorganismen. Die erfindungsgemäßen Substanzen eignen sich daher vorzüglich zum Schutz technischer Materialien.

Technische Materialien in diesem Sinne sind nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, wie beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartons, Leder, Holz, Anstrichmittel, Kunststoffartikel, Kühlschmiermittel und Kühlkreisläufe genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzvefärbende und holzzerstörende Pilze (Basidiomyceten) sowie gegen Schleimorganismen und Algen.

Es seien beispielsweise Mikroorganismen der folgenden Gattungen genannt:

Alternaria, wie Alternaria tenuis,
Aspergillus, wie Aspergillus niger;
Achaetomium, wie Chaetomium globosum;
Coniophora, wie Coniophora puteana;
Lentinus, wie Lentinus tigrinus;
Penicillium, wie Penicillium glaucum;
Polyporus, wie Polyporus versicolor;
Aureobasidium, wie Aureobasidium pullulans;
Sclerophoma, wie Sclerophoma pityophila;
Trichoderma, wie Trichoderma viride;

Escherichia, wie Escherichia coli;
Pseudomonas, wie Pseudomonas aeruginosa;
Staphylococcus, wie Staphylococcus aureus.

Je nach Anwendungsgebiet kann ein erfindungsgemäßer Wirkstoff in die üblichen Formulierungen überführt werden, wie Lösungen, Emulsionen, Suspensionen, Pulver, Pasten oder Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Fall der Benutzung von Wasser als Streckmittel organische Lösungsmittel wie Alkohole als Hilfsmittel verwendet werden können. Flüssige Lösungsmittel für die Wirkstoffe können beispielsweise Wasser, Alkohole, vorzugsweise Ethanol oder Isopropanol oder Benzylalkohol, Ketone, wie Aceton oder Methylethylketon, flüssige Kohlenwasserstoffe, wie Benzinfraktionen, halogenierte Kohlenwasserstoffe, wie 1,2-Dichlorethan, sein.

Mikrobizide Mittel enthalten die Wirkstoffe im allgemeinen in einer Menge von 1 bis 95 %, bevorzugt von 10 bis 75 %.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäßen Wirkstoffe können auch in Mischung mit anderen bekannten Wirkstoffen vorliegen. Beispielsweise seien die folgenden Wirkstoffe genannt: Benzylalkoholmono(oder -poly)hemiformal und andere Formaldehyd abspaltende Verbindungen, Benzimidazolyl-methylcarbamate, Tetramethyldiuramdisulfid, Zinksalze von Dialkyldithiocarbamaten, 2,4,5,6-Tetrachlorisophthalonitril, Thiazolylbenzimidazol, Mercaptobenzthiazol, Organo-Zinnverbindungen, Methylenbisthiocyanat, Phenolderivate, wie 2-Phenylphenol, (2,2'-Dihydroxy-5,5'-dichlor)-diphenylmethan, 3-Methyl-4-chlorphenol, 2-Thiocyanatomethylthiobenzthiazol, N-trihalogenmethylthioverbindungen, wie Folpet, Fluorfolpet und Dichlofluanid, Azolfungizide, wie Triadimefon, Triadimenol, Bitertanol, Tebuconazol, Propiconazol, Azoconazol, Iodpropargylderivate, wie Iodpropargylbutylcarbamat und Iodpropargylphenylcarbamat, Iodthiazolinonverbindungen, wie Kathon sowie quartäre Ammoniumverbindungen, wie Benzalkoniumchlorid.

Ebenfalls können Mischungen der erfindungsgemäß zu verwendenden Substanzen mit bekannten Insektiziden Anwendung finden. Beispielhaft seien hier genannt: Organophosphorverbindungen, wie Chlorpyriphos oder Phoxim, Carbamate, wie Aldicarb, Carbosulfan oder Propoxur oder Pyrethroide, wie Permethrin, Cyfluthrin, Cypermethrin, Deltarnethrin oder Fenvalerat.

Ebenso kommen als Mischpartner Algizide, Molluskizide sowie Wirkstoffe gegen "sea animals", die sich auf Schiffsbodenanstrichen ansiedeln, infrage.

Herstellungsbeispiele:

Beispiel 1:

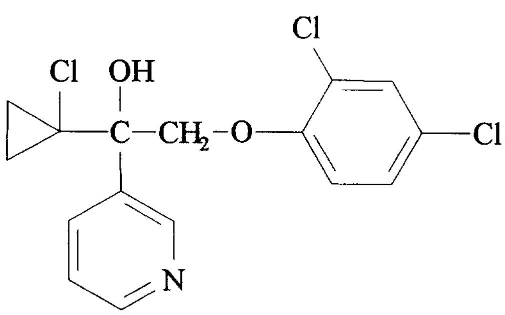

15,8 g (0,1 Mol) 3-Brompyridin - gelöst in einem Gemisch aus 80 ml Diethylether und 80 ml Tetrahydrofuran - werden bei -80 °C tropfenweise unter Rühren unter einer trockenen Stickstoffatmosphäre mit einer Lösung von 30,6 g (0,1 Mol) n-Butyllithium in n-Hexan (23%) versetzt. Nach beendeter Zugabe rührt man weitere 20 Minuten bei dieser Temperatur und gibt dann ebenfalls bei -80 °C eine Lösung von 28,0 g (0,1

Mol) 1-Chlorcyclopropyl-2-(2,4-dichlorphenoxy)-ethan-1-on in 100 ml Tetrahydrofuran zu, rührt anschließend weitere 30 Minuten bei -80 °C, läßt dann die Reaktionsmischung im Verlauf von 2 Stunden auf Raumtemperatur kommen, gibt 140 ml gesättigte wässrige Ammoniumchlorid-Lösung zu, trennt die organische Phase ab, wäscht zweimal mit Wasser, trocknet über Natriumsulfat und entfernt das Lösungsmittel im Vakuum. Der Rückstand wird durch Chromatographie an Kieselgel (Laufmittel: Diethylether) gereinigt.

Man erhält 18,3 g (51 % der Theorie) an 1-(1-Chlorcyclopropyl)-1-(3-pyridyl)-2-(2,4-dichlorphenoxy)-ethanol als Öl.

$^1$H-NMR ($CDCl_3$/Tetramethylsilan): d = 3,68 ppm (1H)

Herstellung der Ausgangsverbindung:

Beispiel II-1:

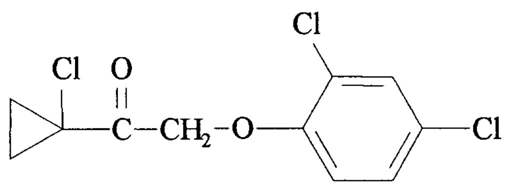

15,3 g (0,1 Mol) 1-Chlorcyclopropyl-2-chlor-ethan-1-on (vergl. z.B. EP 297 383), 18 g (0,11 Mol) 2,4-Dichlorphenol und 16,5 g (0,12 Mol) Kaliumcarbonat werden in 100 ml Toluol 8 Stunden unter Rühren auf Rückflußtemperatur erhitzt. Zur Aufarbeitung wird die abgekühlte Reaktionsmischung in Wasser gegeben, die organische Phase abgetrennt, dreimal mit 10-prozentiger Natronlauge gewaschen, über Natriumsulfat getrocknet, im Vakuum eingeengt und der Rückstand ohne zusätzliche Reinigung direkt weiter umgesetzt.

Man erhält 25,8 g (92 % der Theorie) an 1-Chlorcyclopropyl-2-(2,4-dichlorphenoxy)-ethan-1-on als Öl.

Beispiel 2:

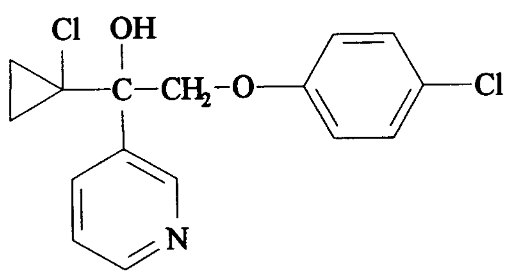

In eine Lösung von 23,8 g (0,162 Mol) Isopropylmagnesiumbromid in 140 ml Diethylether läßt man bei Raumtemperatur unter Rühren 25,6 g (0,162 Mol) 3-Brompyridin - gelöst in 140 ml Tetrahydrofuran - einlaufen. Dabei erwärmt sich die Reaktionsmischung bis zur Rückflußtemperatur. Nach beendeter Zugabe rührt man weitere 30 Minuten bei dieser Temperatur und gibt dann tropfenweise unter Röhren eine Lösung von 33,0 g (0,135 Mol) 1-Chlorcyclopropyl-2-(4-chlorphenoxy)-ethan-1-on in 100 ml Tetrahydrofuran zu, rührt anschließend weitere 30 Minuten bei Rückflußtemperatur, läßt dann die Reaktionsmischung auf Raumtemperatur abkühlen, gibt 50 ml Wasser zu, stellt mit verdünnter Salzsäure einen pH-Wert von 5-6 ein, trennt die organische Phase ab, wäscht zweimal. mit Wasser, trocknet über Natriumsulfat und entfernt das Lösungsmittel im Vakuum Der Rückstand wird durch Chromatographie an Kieselgel (Laufmittel: Diethylether) gereinigt

Man erhält 10,6 g (24 % der Theorie) an 1-(1-Chlorcyclopropyl)-1-(3-pyridyl)-2-(4-chlorphenoxy)-ethanol al Öl vom Brechungsindex $n_D^{20}$ = 1,5665.

Beispiel II-2:

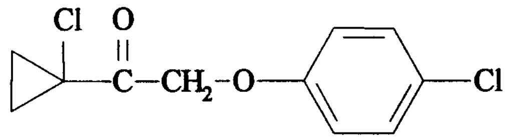

50,4 g (0,392 Mol) 4-Chlorphenol und 54,1 g (0,392 Mol) Kaliumcarbonat werden in 400 ml Toluol 2 Stunden unter Rühren an einem Wasserabscheider auf Rückflußtemperatur erhitzt. Anschließend gibt man bei 100 °C 50,0 g (0,327 Mol) 1-Chlorcyclopropyl-2-chlor-ethan-1-on (vergl. z.B. EP 297 383) zu und rührt weitere 6 Stunden bei 100 °C. Zur Aufarbeitung wird die abgekühlte Reaktionsmischung in Wasser gegeben, die organische Phase abgetrennt, zweimal mit jeweils 200 ml 10-prozentiger Natronlauge und anschließend mit Wasser gewaschen, über Natriumsulfat getrocknet und im Vakuum eingeengt.

Man erhält 66,0 g (82 % der Theorie) an 1-Chlorcyclopropyl-2-(4-chlorphenoxy)-ethan-1-on vom Schmelzpunkt 78 °C.

In entsprechender Weise und gemäß den allgemeinen Angaben zur Herstellung erhält man die folgenden substituierten Hydroxyalkylpyridine der Formel (I):

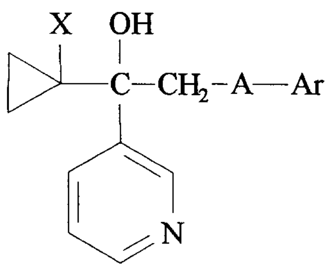

(I)

| Bsp. Nr. | X | A | Ar | physikalische Eigenschaften |
|---|---|---|---|---|
| 3 | Cl | O | 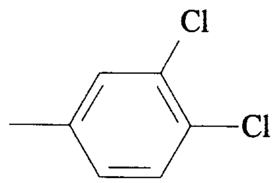 | $^1$H-NMR*): 4,37; 4,4; 4,71; 4,74 (q, 2H) |
| 4 | F | O | 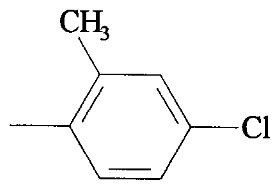 | $^1$H-NMR*): 2,04 (3H) |
| 5 | F | O | 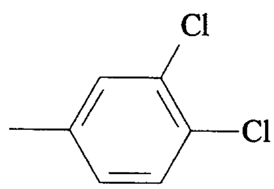 | $^1$H-NMR*): 3,65 (1H) |
| 6 | Cl | O | 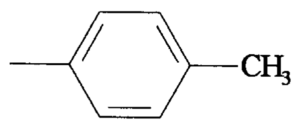 | $^1$H-NMR*): 2,28 (3H) |

| Bsp. Nr. | X | A | Ar | physikalische Eigenschaften |
|---|---|---|---|---|
| 7 | Cl | S | 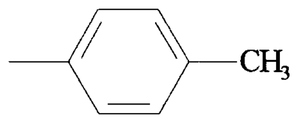 | $^1$H-NMR*): 2,31 (3H) |
| 8 | Cl | O | 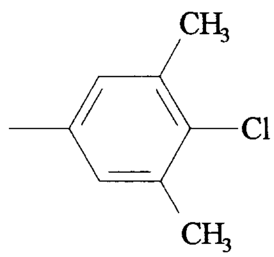 | $^1$H-NMR*): 2,21 (3H); 2,24 (3H) |
| 9 | Cl | O | 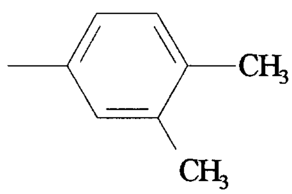 | $^1$H-NMR*): 2,21 (3H); |
| 10 | Cl | O | 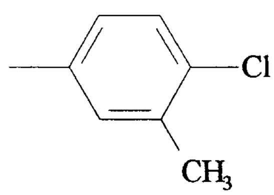 | $^1$H-NMR*): 2,21 (3H); 2,24 (3H) |
| 11 | Cl | S | 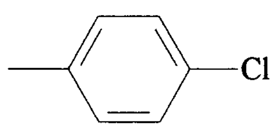 | $^1$H-NMR*): 3,69; 3,37; 3,96; 4,01 (q, 2H) |
| 12 | Cl | S | 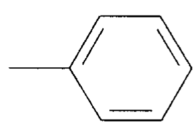 | $^1$H-NMR*): 3,73; 3,77; 3,99; 4,04 (q, 2H) |
| 13 | Cl | O | 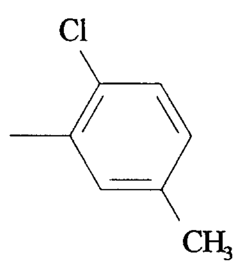 | $^1$H-NMR*): 2,40 (3H); |

| Bsp. Nr. | X | A | Ar | physikalische Eigenschaften |
|---|---|---|---|---|
| 14 | Cl | O | 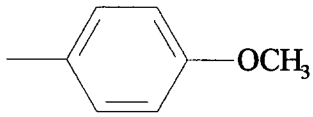 | $^1$H-NMR*): 2,69; 3,71 (3H); |
| 15 | Cl | O | 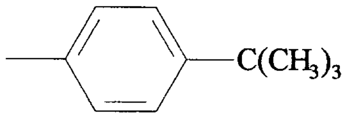 | $^1$H-NMR*): 4,39; 4,43; 4,7; 4,74 (q, 2H); 1,30 (9H) |
| 16 | Cl | O | 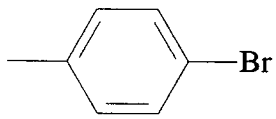 | $^1$H-NMR*): 4,37; 4,4; 4,69; 4,72 (q, 2H) |
| 17 | Cl | O | 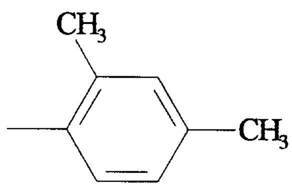 | $^1$H-NMR*): 2,02 (3H); |
| 18 | Cl | O | 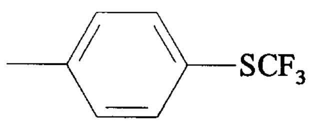 | $^1$H-NMR*): 4,4; 4,43; 4,76; 4,79 (q, 2H); |
| 19 | Cl | O | 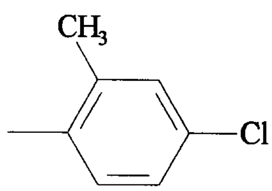 | $^1$H-NMR*): 2,04 (3H); |
| 20 | Cl | O | 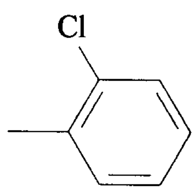 | $^1$H-NMR*): 4,61 (2H); |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als d-Wert in ppm.

Anwendungsbeispiele:

In den folgenden Anwendungsbeispielen wurde die nachstehend aufgeführte Verbindung als Vergleichssubstanz eingesetzt:

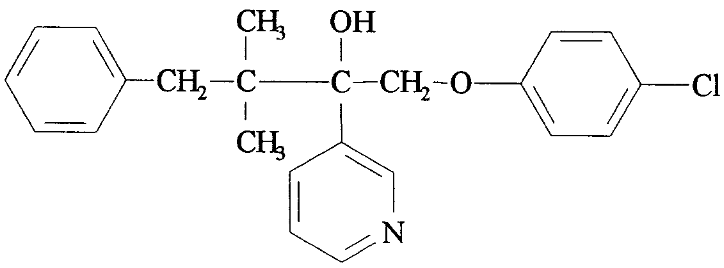

(A)

4-(4-Chlorphenoxy)-2,2-dimethy-3-hydroxy-1-phenyl-3-(3-pyridyl)-butan (bekannt aus EP 302 366)

Beispiel A:

**Erysiphe-Test (Gerste) / protektiv**

Lösungsmittel: 100 Gewichtsteile Dimethylformamid
Emulgator: 0,25 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Bei einer Wirkstoffkonzentration von 250 ppm in der Spritzbrühe zeigen die Verbindungen gemäß den Herstellungsbeispielen 1, 2, 6, 10, 11, 18, 19 und 20 einen Wirkungsgrad von 100 %, während der Wirkungsgrad für die Vergleichssubstanz (A) 50 % beträgt.

Beispiel B:

**Leptosphaeria nodorum-Test (Weizen) / protektiv**

Lösungsmittel: 100 Gewichtsteile Dimethylformamid
Emulgator: 0,25 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung.

Bei einer Wirkstoffkonzentration von 100 ppm in der Spritzbrühe zeigen die Verbindungen gemäß der Herstellungsbeispiele 2 und 6 einen Wirkungsgrad von 100 %, während der Wirkungsgrad für die Vergleichssubstanz (A) 75 % beträgt.

Beispiel C:

**Uncinula-Test (Rebe) / protektiv**

Lösungsmittel: 4,7 Gewichtsteile Aceton
Emulgator: 0,3 Gewichtsteile Alkyl-Aryl-Polyglykolether

Zur Herstellung einer zweckmäßigen Wirkstoffzubereitung vermischt man ein Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Uncinula necator bestäubt.

Die Pflanzen werden anschließend im Gewächshaus bei 23°C bis 24°C und einer relativen Luftfeuchtigkeit von ca. 75 % aufgestellt.

14 Tage nach der Inokulation erfolgt die Auswertung.

Bei einer Wirkstoffkonzentration von 25 ppm in der Spritzbrühe zeigen die Verbindungen gemäß den Herstellungsbeispielen 1, 2, 3, 6, 14, 15, 16, 17, 18 und 19 einen Wirkungsgrad von mindestens 96 %.

**Patentansprüche**

**1.** Substituierte Hydroxyalkylpyridine der Formel (I),

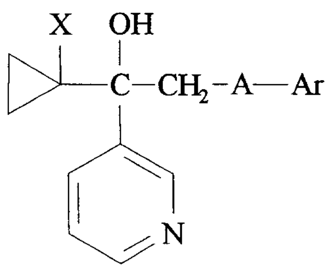

(I)

in welcher

X für Halogen steht,

A für Sauerstoff oder Schwefel steht und

Ar für gegebenenfalls substituiertes Aryl steht,

sowie deren Säureadditions-Salze und Metallsalz-Komplexe.

**2.** Substituierte Hydroxyalkylpyridine gemäß Anspruch 1 der Formel (I), bei welchen

X für Fluor, Chlor, Brom oder Iod steht,

A für Sauerstoff oder Schwefel steht und

Ar für gegebenenfalls einfach bis mehrfach, gleich oder verschieden substituiertes Aryl mit 6 bis 10 Kohlenstoffatomen steht, wobei als Substituenten jeweils infrage kommen:

Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, Cycloalkyl mit 3 bis 8 Kohlenstoffatomen sowie gegebenenfalls einfach bis mehrfach, gleich oder verschieden durch Halogen, geradkettiges oder verzweigtes Alkyl, geradkettiges oder verzweigtes Alkoxy, geradkettiges oder verzweigtes Halogenalkyl oder geradkettiges oder verzweigtes Halogenalkoxy mit jeweils 1 bis 4 Kohlenstoffatomen und gegebenenfalls 1 bis 9 gleichen oder verschiedenen Halogenatomen substituiertes Phenyl.

**3.** Substituierte Hydroxyalkylpyridine gemäß Anspruch 1 der Formel (I), bei welchen

X für Fluor, Chlor oder Brom steht,

A für Sauerstoff oder Schwefel steht und

Ar für gegebenenfalls einfach bis fünffach, gleich oder verschieden substituiertes Aryl mit 6 oder 10 Kohlenstoffatomen steht, wobei als Substituenten jeweils infrage kommen:

Halogen, Cyano, Nitro, jeweils geradkettiges oder verzweigtes Alkyl, Alkoxy, Alkylthio, Alkylsulfinyl oder Alkylsulfonyl mit jeweils 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen

Halogenatomen, jeweils geradkettiges oder verzweigtes Alkoxycarbonyl oder Alkoximinoalkyl mit jeweils 1 bis 3 Kohlenstoffatomen in den einzelnen Alkylteilen, Cycloalkyl mit 3 bis 7 Kohlenstoffatomen sowie gegebenenfalls einfach bis fünffach, gleich oder verschieden durch Halogen, geradkettiges oder verzweigtes Alkyl, geradkettiges oder verzweigtes Alkoxy, geradkettiges oder verzweigtes Halogenalkyl oder geradkettiges oder verzweigtes Halogenalkoxy mit jeweils 1 bis 3 Kohlenstoffatomen und gegebenenfalls 1 bis 7 gleichen oder verschiedenen Halogenatomen substituiertes Phenyl.

**4.** Substituierte Hydroxyalkylpyridine gemäß Anspruch 1 der Formel (I), bei welchen

X für Fluor oder Chlor steht,

A für Sauerstoff oder Schwefel steht und

Ar für gegebenenfalls einfach bis fünffach, gleich oder verschieden substituiertes Phenyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Ethylsulfinyl, Methylsulfonyl, Ethylsulfonyl, Trifluormethyl, Difluormethyl, Trifluormethoxy, Difluormethoxy, Trifluormethylthio, Trifluormethylsulfinyl, Trifluormethylsulfonyl, Methoxycarbonyl, Ethoxycarbonyl, Methoximinomethyl, Methoximinoethyl, Ethoximinomethyl, Ethoximinoethyl, Cyclopropyl, Cyclopentyl, Cyclohexyl oder gegebenenfalls ein- bis dreifach, gleich oder verschieden durch Fluor, Chlor, Brom, Methyl, Ethyl, n- oder i-Propyl, Methoxy, Ethoxy, Trifluormethyl oder Trifluormethoxy substituiertes Phenyl.

**5.** Verfahren zur Herstellung von substituierten Hydroxyalkylpyridinen der Formel (I),

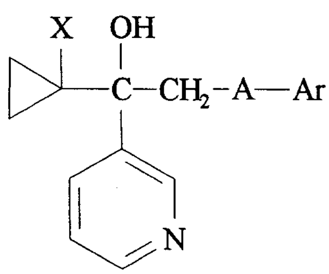

(I)

in welcher

X für Halogen steht,

A für Sauerstoff oder Schwefel steht und

Ar für gegebenenfalls substituiertes Aryl steht,

sowie von deren Säureadditions-Salzen und Metallsalz-Komplexen, dadurch gekennzeichnet, daß man Cyclopropylketone der Formel (II),

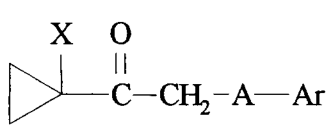

(II)

in welcher

X, A und Ar die oben angegebenen Bedeutungen haben,

mit metallorganischen Pyridin-Verbindungen der Formel (III),

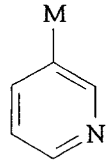

(III)

in welcher

M für ein Lithiumatom oder für eine Magnesium-Halogen-Gruppierung steht,

gegebenenfalls in Gegenwart eines Verdünnungsmittels umsetzt und gegebenenfalls an die so erhaltenen Verbindungen der Formel (I) eine Säure oder ein Metallsalz addiert.

**6.** Schädlingsbekämpfungsmittel, gekennzeichnet durch einen Gehalt an mindestens einem substituierten Hydroxyalkylpyridin der Formel (I) nach den Ansprüchen 1 und 5 bzw. an einem Säureadditions-Salz oder Metallsalz-Komplex eines substituierten Hydroxyalkylpyridins der Formel (I).

**7.** Verwendung von substituierten Hydroxyalkylpyridinen der Formel (I) nach den Ansprüchen 1 und 5 bzw. von deren Säureadditions-Salzen oder Metallsalz-Komplexen zur Bekämpfung von Schädlingen.

**8.** Verfahren zur Bekämpfung von Schädlingen, dadurch gekennzeichnet, daß man substituierte Hydroxyalkylpyridine der Formel (I) nach den Ansprüchen 1 und 5 bzw. deren Säureadditions-Salze oder Metallsalz-Komplexe auf Schädlinge und/oder ihren Lebensraum einwirken läßt.

**9.** Verfahren zur Herstellung von Schädlingsbekämpfungsmitteln, dadurch gekennzeichnet, daß man substituierte Hydroxyalkylpyridine der Formel (I) nach den Ansprüchen 1 und 5 bzw. deren Säureadditions-Salze oder Metallsalz-Komplexe mit Streckmitteln und/oder oberflächenaktiven Mitteln vermischt.

Europäisches Patentamt

# EUROPÄISCHER RECHERCHENBERICHT

Nummer der Anmeldung
EP 93 11 3475

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,Y | EP-A-0 302 366 (BAYER AG) 8. Februar 1989 * Seite 40, Zeile 15 - Seite 40, Zeile 26; Ansprüche 1-8 * | 1-9 | C07D213/30 C07D213/32 C07D213/34 C07D213/26 A01N43/40 |
| Y | DE-A-2 742 173 (BAYER AG) 29. März 1979 * Seite 7, Zeile 28 - Seite 8, Zeile 23; Ansprüche 1-6 * | 1-9 | |
| Y | DE-A-3 250 138 (CONSORTIUM FÜR ELEKTROCHEMISCHE INDUSTRIE GMBH) 30. Juni 1983 * Seite 15, Zeile 17 - Seite 16, Zeile 2; Ansprüche 1-4 * | 1-9 | |
| Y | CH-A-620 910 (ELI LILLY AND CO.) 31. Dezember 1980 * Seite 2, rechte Spalte, Zeile 55 - Zeile 61; Ansprüche 1-9 * | 1-9 | |
| | | | RECHERCHIERTE SACHGEBIETE (Int. Cl.5 ) |
| | | | C07D A01N |

Der vorliegende Recherchenbericht wurde für alle Patentansprüche erstellt

| Recherchenort | Abschlußdatum der Recherche | Prüfer |
|---|---|---|
| MUENCHEN | 13 JANUAR 1994 | HERZ C.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument